# EUROPEAN PATENT SPECIFICATION

(11) **EP 2 510 358 B1**
(45) Date of publication and mention of the grant of the patent: **16.07.2014**
(21) Application number: 10836433.2
(22) Date of filing: 30.11.2010
(51) Int. Cl.: A61P 35/04, A61K 38/00

(54) **Oxidised streptolysin for use in the treatment of cancer metastasis**
Oxidiertes Streptolysin zur Verwendung bei der Behandlung von Krebsmetastasen
Streptolysin oxydé pour son utilisation dans le traitement la métastase cancéreuse

(30) Priority: 07.12.2009 US 632149
(43) Date of publication of application: 17.10.2012
(73) Proprietor: Beech Tree Labs Inc., Delanson, NY 12053 (US)
(72) Inventor: MCMICHAEL, John, Delanson NY 12053 (US)
(74) Representative: Patentanwälte Olbricht Buchhold Keulertz
(86) International application number: PCT/US2010/058421
(87) International publication number: WO 2011/071722

(56) References cited:
- WO-A1-2007/049924
- US-A1- 2008 182 789
- US-A1- 2008 182 789
- US-A1- 2009 202 587
- ELENA I DERYUGINA ET AL: "Matrix metalloproteinases and tumor metastasis", CANCER AND METASTASIS REVIEWS, vol. 25, no. 1, 1 March 2006 (2006-03-01), pages 9-34, XP019392609, KLUWER ACADEMIC PUBLISHERS, DO ISSN: 1573-7233, DOI: 10.1007/S10555-006-7886-9
- TOMIC-CANIC M ET AL: "Streptolysin O enhances keratinocyte migration and proliferation and promotes skin organ culture wound healing in vitro", WOUND REPAIR AND REGENERATION, vol. 15, no. 1, 1 January 2007 (2007-01-01), pages 71-79, XP002531948, MOSBY-YEAR BOOK, ST. LOUIS, MO, US ISSN: 1067-1927, DOI: 10.1111/J.1524-475X.2006.00187.X
- SHAILANDER BATHIA ET AL.: "Treatment of Metastatic Melanoma: An Overview", NIH Public Access Author Manuscript , May 2009 (2009-05), XP002697682, Retrieved from the Internet: URL:http://www.ncbi.nlm.nih.gov/pmc/articl es/PMC2737459/pdf/nihms129601.pdf [retrieved on 2013-06-12]
- "Principles of cancer Therapy ED - BEERS M H (; ET AL) (EDS)", 1 January 2006 (2006-01-01), THE MERCK MANUAL OF DIAGNOSIS AND THERAPY, MERCK RESEARCH LABORATORIES, WHITEHOUSE STATION, NJ, XP002695786, ISBN: 978-0-911910-18-6 pages 1157-1171, * the whole document *
- EMILY H HALL ET AL: "Inhibition of human breast cancer Matrigel invasion by Streptolysin O activation of the EGF receptor ErbB1", CELLULAR SIGNALLING, vol. 23, no. 12, 5 July 2011 (2011-07-05), pages 1972-1977, XP028303636, ELSEVIER SCIENCE LTD, GB ISSN: 0898-6568, DOI: 10.1016/J.CELLSIG.2011.07.007 [retrieved on 2011-07-20]

## Description

Herein described is the treatment of various conditions by the administration of streptolysin O including connective tissue disorders, reproductive fibroses and conditions mediated by the CD44 receptor. Herein described are means for protecting nerve cells from the effects of neurotoxic agents by the administration of streptolysin O. Herein described as means of alleviating symptoms of a connective tissue disorder, such as tendonitis, by the administration of streptolysin O. The present invention is described in claims 1-17.

### BACKGROUND OF THE INVENTION

Connective tissue is the material between the cells of the body that gives tissues form and strength. This "cellular glue" is also involved in delivering nutrients to the tissue, and in the special functioning of certain tissues. Connective tissue is made up of dozens of proteins, including collagens, proteoglycans, and glycoproteins. The combination of these proteins can vary between tissues. The genes that encode these proteins can harbor defects or mutations, which can affect the functioning of certain properties of connective tissue in selected tissues. As described below, there are a number of different disease states where connective tissue play an important role in the pathological manifestations of the particular disease including Dupuytren's contracture, scleroderma, Peyronie's disease, claudication due to peripheral arterial disease and mastitis in animals.

Herein described are means for treatment of connective tissue disorders including Dupuytren's contracture, scleroderma, Peyronie's disease, and lower limb claudication. These diseases take an enormous toll on people's ability to work, perform physical and sexual activity, maintain normal living standards, and perform everyday activities. In addition, post chronic mastitis infections in bovines have huge economic implications on the viability of livestock and the food supply, specifically the dairy industry.

Peripheral arterial disease (PAD) involves damage to or blockage of the blood vessels distant from the heart (usually in the arms and the legs) and includes several clinical syndromes in the extremities characterized by pain, inflammation, and ischemic damage to soft tissues from partial or complete occlusion of major arteries. The most characteristic symptom of PAD is intermittent claudication, which is described as cramping, aching, and numbness of the extremities induced by exercise. Intermittent claudication subsides by ending the exercise regiment. The symptoms of claudication result from atherosclerosis, which is a condition where plaque consisting of cholesterol, fats, calcium, and fibrin (blood clotting agent) build up on the inside of the artery wall. The artery wall consists of three layers: a layer of connective tissue, a second layer of smooth muscle cells and elastic connective tissue, and a third layer of endothelial cells. Damage to these cells leads to thrombocyte adhesion, aggregation, and formation of thrombi or intima in the arterial wall. This formation allows monocytes to stick to the arterial wall and maturate into macrophages, while recruiting LDL cholesterol to create a foam cell formation or fatty streaks. This interruption in the arterial endothelial lining causes platelets to become activated and recruit smooth medial muscle cells into the initma leading to connective tissue proliferation and lipid uptake. This cycle of inflammation and proliferation of connective tissue in the arterial wall of the blood vessel leads to narrowing of the arterial lumen, restricting blood flow.

The risk factors for atherosclerosis in the peripheral arteries of the legs and arms are the same as those for atherosclerosis in the coronary arteries. Smoking, diabetes, high blood pressure, and high cholesterol lead to the development of plaque. Most people with atherosclerosis in the leg arteries have no symptoms because the body develops small blood vessels (collateral vessels) around the blockage. With sustained activity, the collateral vessels are unable to supply enough oxygen to the leg's muscles and therefore, the pain is associated in the calf, thigh or buttocks muscles. In more advanced claudication, pain can occur even while one is at rest. If this symptom is left untreated, the lack of circulation may result in sores on the legs and feet, and the tissue can become gangrenous, requiring amputation.

Claudication is often a sign of atherosclerosis of both the coronary and carotid arteries. In treating atherosclerotic diseases, physicians should focus on evaluation, risk factor modification (quitting smoking and reducing cholesterol), and exercise (stimulate carotid arteries and condition muscles) (see Carmen et al., Am Fam. Physician 61:1027-1034 (2000); Gardner et al., JAMA 274:975-980 (1995); Patterson et al., J Vasc. Surg. 25:312-319 (1997)). Antiplatelet agents, which prevent the recruiting activities of platelet cells, such as aspirin, ticlopidine, or clopidogrel, reduce the risk of vascular death, myocardial infarction, and stroke as much as 24% (see Goldhaber et al., Lancet 340:143-145 (1992); Janzon et al., J. Intern. Med. 227:301-308 (1990); Lancet 348:1329-1339 (1996)). The combination of exercise and the drug pentoxifylline appears to reduce claudication (Hood et al., CMAJ 155:1053-1059 (1996)). The drug, Cilostazol, a phosphodiesterase inhibitor that suppresses platelet aggregation and arterial vasodilator, increases the amount and quality of exercising a patient can perform to overcome claudication (Dawson et al., Circulation 98:678-686 (1998)). Although these medical measures show some improvement of claudication, there remains a need in the art for methods to better treat peripheral arterial disease.

Dupuytren's contracture is a painless thickening and contracture of tissue beneath the skin on the palm of the hand. The cause of the contracture is unknown, but minor trauma and genetic predisposition may play a role. One or both hands may be affected. The ring finger is affected most often, followed by the little, middle and index finger. A small, painless nodule develops in the connective tissue and eventually develops into a cord-like band. Gradually, other nodules may develop and extend a contracture across the first joint into the finger. The overlying skin begins to pucker, and rough cords of tissue extend into the finger. As the process continues, these cords tighten and pull the finger in toward the palm. The ring finger is usually affected first, followed by the little, long and index fingers. The problem is not pain, but the restriction of motion and the deformity it causes. The progress of the disease is often sporadic and unpredictable. Exactly what triggers the formation of nodules and cords is unknown. As the disease progresses, the diseased nodules wraps itself around and between the normal tissue.

The incidence increases after the age of 40, and men are affected more often than women. Interestingly, the risk factors are associated with alcoholism, epilepsy, pulmonary tuberculosis, diabetes, and liver disease. Treatment for this disease can include exercises to stretch the diseased tissue, warm water baths, or splints. Often, these measures only slow the contracture, but do not cure the contracture. If the contracture continues, surgery may be performed to release the contracture, depending upon the severity of the condition. Several techniques including fasciectomy, dermofasciectomy, fasciotomy, and amputation are used. Fasciectomy is a corrective surgery performed by removing the fascia tissue and stitching up the wound in a zigzag manner (See Visa et al., Romanian Journal of Hand and Reconstrictive Microsurgy 5:9-13 (2000)). Dermofasciectomy is a corrective procedure of an advanced state of Dupuytren's contracture, where the skin and the fascia bands and nodules are removed. The removed skin is replaced by a skin graft. Fasciotomy is a medical procedure for elderly patients unfit for complicated surgery where the bands are cut. Finally, in rare cases, fingers in which the bands have returned many times and previous nerve and tissue damage exist, amputation of the finger is an option. While surgery usually restores normal movement to the fingers, the disease can reoccur following surgery and the risk of nerve damage increases after each surgery. Therefore, there remains a need for a less drastic method for treating Dupuytren's contracture.

Peyronie's disease is a disorder of the connective tissue within the penis that can cause curvature during erection. The disease is characterized by a plaque, or a hard lump, that forms in the erectile tissue of the penis. It begins as a localized inflammation and can then mature into a hardened scar. The cause of Peyronie's disease can be attributed to the septum connective tissue, which lines the inner membrane of each erectile cylinder that runs the length of the penis and attaches at the top and bottom of the penis. If the penis is abnormally squeezed or flexed, the area where the septum attaches to the elastic fibers may over-stretch, injuring the lining of the erective chamber and rupturing small blood vessels. In older men, diminished elasticity, disease of the arteries, diabetes, or radical prostatectomy further increase the chance of injury. In fact, Peyronie's disease is diagnosed in only 26 out of 100,000 men each year; however, the ratio increases to 3 out of 64 patients who develop Peyronie's disease after a radical prostatectomy (Jarrow et al., J. of Urology 158:1388-1390 (1997)).

Men with Peyronie's disease usually seek medical attention because of painful erections or difficulty with intercourse. The goal of any treatment is to keep the Peyronie's patient sexually active. Providing education about the disease and its course is often all that is required. There is no strong evidence that any treatments other than surgery are effective. Experts usually recommend surgery only in long-term cases where the disease has stabilized and where the deformity prevents intercourse. The two most common surgical methods are removal or expansion of the plaque followed by a placement of a patch of skin or artificial material and removal of pinching tissue from the side of the penis. Both procedures have the disadvantage of side effects including loss of erectile function or shortening of the erect penis. Often, the plaques of Peyronie's disease shrinks or disappears without treatment over a 6-15 month period, and thus, medical experts suggest waiting 1 to 2 years before attempting to correct it surgically. Spontaneous improvement in the disease is seen in 60-70% of patients.

Simple medical treatments have not been clinically proven. Some researchers have given men with Peyronie's disease vitamin E orally in small-scale studies, but these studies have proven inconclusive. Also, similar inconclusive success has been attributed to oral application of para-amino benzoate, a substance belonging to the family of B-complex molecules. Injection treatment with agents such as dimethyl sulfoxide, steroids, and calcium channel blockers directly into the plaques is used by some doctors, but none of these techniques have produced convincing results. The only medical treatment proven to be effective is Tamoxifen, which can relieve the pain and limit any subsequent bending of the penis. The disadvantage of Tamoxifen is that Peyronie's disease must be diagnosed early for the most effective use of the drug and therefore, there remains a desire in the art for methods for the treatment and prevention of Peyronie's disease.

Scleroderma is an autoimmune disease of the connective tissue, which affects many body systems such as the gastrointestinal tract, the respiratory, renal, cardiovascular, and genitourinary systems, but is primarily characterized by thickening and tightening of the skin. This disease may either be visible, as when the skin is affected, or invisible, as when only internal organs are involved, but is usually a highly-individualized disease wherein its involvement may range from mild symptoms to life-threatening symptoms. The symptoms result from progressive tissue fibrosis and occlusion of the microvasculature by excessive production and deposition of types I and II collagens. Other macromolecules found in connective tissue (e.g., glycosaminoglycans, tenascin, fibronectin) increase in production due to inflammation of the area experiencing fibrosis. The vascular alternations show a predilection for affecting the small arteries, arterioles, and capillaries. The small vessel cytoskeleton is affected by structural defects that lead to collapse. Next, the tight junctions become altered and are no longer functional allowing the endothelium to slip into the vessel lumen.

An estimated 300,000 persons in the United States have scleroderma with more women (4 times more) than men developing the disease usually between the ages of 20 to 50. Symptoms of scleroderma include one or more of the following: Raynaud's Phenomenon (abnormal sensitivity to cold in the extremities), swelling of the hands and feet, pain and stiffness of the joints, thickening of the skin, joint contractures, digestive system and gastrointestinal tract problems, Sjogren's Syndrome (dry mucus membranes), oral, facial and dental problems, kidney, heart, and lung involvement, and non-specific symptoms such as extreme fatigue, generalized weakness, weight loss, and vague aching of muscles, joints and bones. The most serious side effect of scleroderma is pulmonary hypertension, and its complications are the most frequent causes of mortality. For example, the lungs are affected in 70-80% of patients, and develop either fibrosis or change in the blood vessels, which leads to increased pressure in the pulmonary arteries (Harrison et al., Am. Rev. Respir. Dis. 144:706-713 (1991); Silver et al., Am. J. Med. 88:470-476 (1990)). The fibrosis usually starts with an increase in lung fiber density near the posterior (back) regions of the lungs. Later stages of fibrosis are characterized by the emergence of a network of fibrous lines. These fibrous lines eventually develop into regions containing large numbers of small cysts. The end-stage effect is sometimes referred to as "honeycombing" and is non-reversible (Wallaert et al., Am. Rev. Respir. Dis. 133:574-580(1986)).

The goal for treating scleroderma is to prevent further complications (*i.e*. fibrosis) and reduce morbidity if complications exist. Primary treatment consists of inhibiting the immune system alterations, which may be responsible for the wide variety of systemic morbidity associated with this disease. Skin thickening can be treated with D-penicillamine and methotrexate, which both increases the effects of immunosuppressants and slows down the formation of collagen. The experimental drug relaxin has also shown promise reducing the extent and severity of skin thickening in patients with diffuse scleroderma (Seibold et al., Ann. Intern. Med. 132:871-879 (2000)). Relaxin attenuates the actions of profibrotic cytokines including transforming growth factor-β and interleukin-1β, and increases secretion of dermal fibroblast collagenase, while reducing levels of tissue inhibitor of metalloproteinase (Unemori et al., J. Biol. Chem. 265:10681-10685 (1990)). Raynaud phenomenon can be treated with calcium blockers or topical nitrates. Gastrointestinal symptoms may be treated with antacids, pump inhibitors, and laxatives. More severe complications, like fibrosis in the lungs or pulmonary hypertension, require more drastic measures. For example, scleroderma and alveolitis (hypersensitive inflammation of alveolar cells in the lung) can cause severe damage to lung tissues. Treatment with experimental drugs such as cyclophosphamide work to inhibit inflammation, but is not effective against only scleroderma in the lungs. Rather, both sets of symptoms are required (White et al., Ann. Intern Med. 132:947-954 (2000)). Pulmonary hypertension is a relatively common complication of systemic sclerosis with a lack of viable treatment options and a high mortality rate. In light of these factors, the use of intravenous epoprostenol has shown some promise (Badesch et al., Ann. Intern. Med. 132:425-434 (2000)), but may have limited applicability due to possible acute and potentially fatal side effects such as pulmonary edema in patients suffering with veno-occlusive disease as well as scleroderma (Barst et al., N. Eng. J. Med. 334:296-301 (1996)). Many of these treatments are in their experimental stages, and the current treatments for the various scleroderma complications either cause the patients to experience severe side effects, place them at risks for further complications or require a unique set of symptoms to provide adequate treatment. Thus, there remains a need in the art for improved methods for treating scleroderma.

Of interest is a study which reports that streptolysin O has been shown to modulate or reduce the excessive production of collagen in two murine models of scleroderma (Mamber et al., Nonlinear. Biol. Toxicol. Med., 2:67-87,2004).

Post chronic mastitis infection is a connective tissue disorder that can prevent adequate lactation ofbovines. Mastitis is an inflammation of the udder that affects a high proportion of dairy cows throughout the world. There are three major types of mastitis, corresponding to three distinct stages of development. Acute mastitis is generally characterized by redness, heat, pain, hardness or swelling accompanied by fever, a loss of appetite, and lower milk production. There are two stages during acute mastitis including (1) the inflammatory stage where there is no infection and few to no lumps in the teats; (2) the infection stage where pus is generated and lumps begin to form. Bacteria such as *Escherichia coli, Streptococcus dysgalactiae,* coagulase-negative staphylococci, *Staphylococcus aureus, Streptococcus uberis,* colorless algae and corneybacterium can cause the initial infection via numerous vectors such as flies, flowing water, standing water, water tanks, water runoff from silage, well water, manure, teat dip containers, milking machine liners, teat end swabs and feed troughs. These bacteria are able to invade the mammary gland, multiply therein, and produce harmful substances that result in an inflammatory response. Once infection begins, the teats can become so infected that the milk first becomes yellow and then watery. After infection, chronic mastitis can occur which is the after-effect of repeated bouts of mastitis at the level of the teat where humps, lesions, hardenings, damaged teats, lost quarters, nodularthelitis, and a drop in milk production occur.

The focus of treatment is dependent upon the level of infection and how many repeat occurrences of mastitis have occurred. It is important to diagnose mastitis early in the infection. Chronic mastitis is the most critical to prevent. Animals with chronic mastitis often acquire permanent damage to the teat and the bovine loses productivity and is unable to release milk at a sufficient level from the damaged teat.

Mastitis is difficult to control since several bacteria have the ability to infect the udder. Even well managed dairy herds that utilized the most recent and most effective mastitis control measures witness a high rate of infection in the first 90 days of lactation (Schrick et al., Department of Animal Science Annual Report, The University of Tennessee, Knoxville, in press). Mastitis has been described as the most economically imposing disease facing dairy producers in the United States, costing an estimated $2 billion annually (DeGraves and Fetrow, The Veterinary Clinics of North America-Food Animal Practice Update on Bovine Mastitis 9:421-434 (1993)). Thus, there remains in the art the need for treatment that will allow bovines to continue to exhibit productive milk even after damage to the udder has occurred due to complications from chronic mastitis.

Reproductive fibrosis in the form of fibroids (noncancerous growths) affects the reproductive organs of female mammals, most notably the uterus and the fallopian tubes. Fibroids can grow inside, within the muscle wall and on the outside surface of the uterus. Reproductive fibrosis can result in various symptoms including pain, bleeding, urinary tract, bladder and kidney infection, infertility and difficulties with pregnancy. Reproductive fibroids do not occur prior to puberty and frequently cease having symptoms after the conclusion of menopause. Fibroids that do not cause symptoms or that cause only minor symptoms usually do not require treatment. Reproductive fibroses that cause more severe symptoms are treated with medications such as oral contraceptives or gonadotropin-releasing hormone agonists (GnRH-As). Non-surgical treatments such as uterine fibroid embolization which is a radiological procedure may be carried out to treat fibroids. Further, surgical treatments such as myomectomy (which is intended to retain fertility) or hysterectomy (which will not retain fertility) may be carried out. Despite these therapies, there remains a desire in the art for additional options for treatment of reproductive fibroses.

Of interest to the present invention is the CD44 family of surface receptors which regulate various cellular activities. The CD44 receptor protein is a transmembrane glycoprotein with an approximate molecular weight of about 37 kD and has a role in matrix adhesion lymphocyte activation to the basement membrane and in the maintenance of epithelial cell polarity. As such, the CD44 receptor protein is also known as the lymph node homing receptor and is homologous to the "cartilage link protein." Hyaluronic acid (HA) is one of its ligands. Altered expression of the CD44 receptor is believed to be associated with tumor progression and metastases in various cancers. In addition, degradation of HA may play a critical role in promoting the formation of scar tissue in the damaged nervous system that inhibits axonal regeneration following injury to the brain or spinal cord. In contrast, the accumulation of high molecular weight forms of HA may contribute to the pathogenesis of various neurodegenerative diseases, including multiple sclerosis and Alzheimer's disease. Also of interest to the present invention is the observation that the CD44 receptor on fibrous astrocytes appears to be up-regulated in Multiple Sclerosis. Accordingly, there exists a desire for agents and methods to intervene with the biological activities of the CD44 receptor as well as the effects of hyaluronic acid on that receptor. Further, there also exists a need for neuroprotective agents to protect neurons against both natural and synthetic neurotoxic agents in the environment and which result *in vivo* as a result of metabolic processes.

Streptolysin O is one of a group of filterable hemolysins derived from Group A beta-hemolytic streptococci. Specifically, streptolysin O is a 60-kD peptide, which is hemolytic in its reduced state, but is inactivated upon oxidation. Group A streptococci produce streptolysin O. Streptolysin O is used in the art generally as an analytical reagent for permeabilizing cells (e.g. Razin et al., Proc. Nat'l. Acad. Sci. (USA) 91:7722-7726 (1994). Streptolysin O is hemolytic in its reduced state but is inactivated upon oxidation (Johnson et al., Infect. Immun., 27:97-101, 1980; Alouf et al., Pharmacol. Ther., 3:661-717, 1984; Bhakdi et al., Infect. Immun., 47:52-60, 1985).

It is thought that induction of a pro-inflammatory response in keratinocytes (skin cells) is associated with adherence of streptococci and their production of streptolysin O (Ruiz et al., Mol. Microbiol. 27:337-346 (1997); Cunningham, M.W., Clin. Microbiol. 13:470-511 (2000)). Specifically, the hyaluronic acid capsule of group A streptococci may be an important adherence factor since it binds to CD44 on epithelial cells (Schrager et al., J. Clin. Investig. 101:1708-1716 (1998)). Streptolysin O may also interact with CD-44 receptors on keratinocytes and dissolve collagen to allow streptococci to get in the blood stream. It has recently been reported that streptolysin O enhances keratinocyte migration and proliferation and promotes skin organ culture wound healing in vitro (Tomic-Canic et al., Wound Rep. Reg., 15:71-79, 2007).

The pharmacological effects of low levels of ML-05, a modified form of the hemolytic and cytotoxic streptolysin O, on keratinocyte growth and up-regulation of CD44 were characterized in previous *in vitro* studies (Mamber et al., Nonlinear. Biol. Toxicol. Med., 2:67-87, 2004). Briefly, ML-05 appeared to decrease skin collagen levels in two *in vitro* models of collagen disorders, the tight skin mouse (Tsk) model of scleroderma, and the bleomycin-induced mouse skin fibrosis model.

It has been reported that prior infection with a Group A beta-hemolytic streptococcus is linked to subsequent development of movement disorders. Taranta, et al., Am. J. Med., 20: 170-175 (1956). Moreover, there are reports that patients having Group A beta-hemolytic streptococcal infections produce antibodies against their own neural tissue and that such antibodies are stimulated by the streptococcal infection. Kiessling, et al., Pediatrics, 92:39-43 (1993). Interestingly, patients with central nervous system deficits which result in impaired movement have high anti-streptolysin O antibody titers and those antibodies cross-react with myelin basic protein, a suspected causative agent in multiple sclerosis.

Tendonitis is perhaps one of the most frustrating athletic injuries involving the lower leg of a horse. Tendonitis is defined as the inflammation of the tendon and tendon-muscle attachments. A "bowed tendon" is caused by an injury to the superficial digital flexor tendon, the tendon lying closest to the skin on the abck portion of the leg between the carpus (knee) and the fetlock (ankle). Tendonitis usually results from a severe strain or hyperextension of this tendon during excessive loading during work. Poor footing in the arena, excessive pastern slope, improper shoeing, and tight fitting leg wraps may place extra stresses on this tendon.

Clinical signs include, diffuse swelling on the back of the leg, heat, pain on palpation, and varying degrees of lameness depending on the severity of the injury. Tendon injuries can vary from mild inflammation of the surrounding tendon structures, to more severe tears of the tendon fibers, to partial or complete rupture. An aggressive approach to therapy may make the difference in the horse's ability to return to its previous level of performance. Inappropriate therapy may lead to chronic lameness problems due to re-injury of the tendon. Surgical options include: ultrasound guided tendon puncture and/or superior check ligament desomtomy. The goal of medical treatment is to keep the inflammatory response to a minimum. Ideally the aim is to limit the amount of inflammation to that necessary to repair the injury and keep the inflammation from affecting the remaining normal tendon (reduce unwanted scar tissue). Current therapy includes one or more of the following: cold therapy (application of cold water or ice four to six times a day); bandaging to provide counter pressure against the swelling; stall rest; and anti-inflammatory medication.

Despite significant advances in medical research and technology, cancer continues to be one of the leading causes of death in the United States and throughout the world. There are in excess of one million new cases of cancer reported in the United States alone, and more than half a million people die in this country every year from cancer.

Current treatments for cancer include surgical removal, chemotherapy and/or radiation treatment of tumors, yet each has its limitations. In the former case, once a tumor has metastasized by invading the surrounding tissue or by moving to a distant site, it can be virtually impossible for the surgeon to remove all cancerous cells. Any such cells left behind can continue growing, leading to a recurrence of cancer following surgery. Current radiation therapy strategies are also frequently unsuccessful at eradicating a patient's cancer. Following radiation therapy, cancer can recur because it is often not possible to deliver a sufficiently high dose of radiation to kill all the tumor cells without at the same time injuring the surrounding normal tissue. Cancer can also recur because tumors show widely varying susceptibilities to radiation-induced cell death. Thus, the inability of current treatment protocols to eliminate tumor cells is an important clinical limitation leading to unsuccessful cancer therapy (Lindegaard et al., J. Natl. Cancer Inst. Monogr., 21:105-112, 1996; Suit, Front. Radiat. Ther. Oncol., 29:17-23, 1996)

Newer treatment strategies are needed to address the challenges that result from the inability to successfully treat neoplastic disease. One of the major challenges of cancer treatment is selectivity of the therapy: the ability to kill tumor cells without causing damage to normal cells in the surrounding area. Various current approaches take advantage of the fact that in most cases tumor cells grow more quickly than normal cells, so strategies designed to kill rapidly growing cells are somewhat selective for tumor cells (see Yazawa et al., World J. Surg.. 26:783-789, 2002). These methods, however, also kill certain cell types in the body that normally divide rapidly, most notably cells in the bone marrow, resulting in complications such as anemia and neutropenia (reviewed in Vose & Armitage, J. Clin. Oncol., 13:1023-1035, 1995). Other strategies are based upon the production of antibodies directed against tumor-specific antigens (reviewed in Sinkovics & Horvath, Int. J. Oncol., 16:81-96, 2000).

Of interest to the present application is U.S. Patent Application Publication No. 2008/0286238 which is directed to gene therapy comprising the administration of transgenes encoding streptolysin O to kill transfected cancer cells by expressing the pore-forming toxin and permeabilizing the cellular membrane.

Although the precise mechanisms by which malignant tumor cells metastasize are not fully understood, metastasis and cell invasion are thought to be mediated, at least in part, through the degradation of basement membrane by neutral matrix metalloproteinases (MMP) produced by tumor cells. MMPs are members of a unique family of zinc-binding endopeptidases that together have the ability to mount a concerted degradative attack on virtually all components of the extracellular matrix (ECM). These enzymes are secreted as catalytically latent species that are processed to their activated forms in vivo by other proteinases. Members of this important protease family have been divided into five subclasses based on structural similarity and substrate specificity. These include: collagenases (MMP-1, MMP-8 & MMP-13), gelatinases (MMP-2 and MMP-9), stromelysins (MMP-3 and MMP-10), metalloclastase, Membrane-type MMPs (MT-MMP; MMP-14, MMP 15, MMP-16, MMP-17, MMP-24, and MMP-25), and others (MMP-7, MMP-11, MMP-12, MMP-19, MMP-20 and MMP-23).

The uncontrolled expression of MMPs, especially MMP-2 and -9, is associated with many pro-oncogenic events such as angiogenesis, tumor cell invasion, and tumor cell metastasis. Although few cell types express MMP-9 physiologically, the majority of human metastatic tumor cells (i.e., melanoma, fibrosarcoma, breast adenocarcinoma, glioma, etc.) that have been tested consistently show elevated MMP-9 activity compared with benign control cells. Tumor cells stably expressing the MMP-9 cDNA have been shown to widely metastasize in nude mice, whereas inhibition of the MMP-9 activity has been demonstrated to prevent metastasis in other models.

Interestingly, immunohistochemical examination of benign and malignant breast disease has shown that mRNA and protein for MMPs are expressed not only by the tumor cells but also by cells in the surrounding stroma. These data suggest that the interaction of tumor cells with the basement membrane may trigger the expression and release of MMPs by the surrounding tissues, ultimately resulting in increased degradation of the basement membrane with subsequent metastasis. One of the major components of the poor prognosis associated with high-grade gliomas is their ability to invade tissues and migrate with subsequent metastasis to distant sites in the brain. Many human glioma cell lines have been shown to express MMPS, and MMP2 and - 9 in particular have been shown to be up-regulated at the mRNA and protein levels in the SNB 19 and the U87MG human glioma cell lines. In addition, it has been demonstrated that the rat C6 glioma cell line also expresses MMP2 and -9 at the mRNA and protein level.

The disclosures of co-owned U.S. Patent Nos. 5,576,289 and 5,736,508 disclosures are hereby cited. U.S. Patent No. 5,576,289 discloses the use of streptolysin O in methods for treating disease states characterized by motor deficit including multiple sclerosis and autism. U.S. Patent No. 5,736,508 discloses the use of streptolysin O in methods for treating scarring. No disclosure, however, is made of an utility wherein streptolysin O is used to treat connective tissue disorders such as tendonitis, Dupuytren's contracture, scleroderma, Peyronie's disease, mastitis in animals, and claudication due to peripheral arterial disease, that streptolysin O has direct neuroprotective effects or therapeutic effects on reproductive fibroses or that it has the effect of inhibiting hyaluronic acid binding to CD44 receptors or otherwise inhibits CD44 mediated processes. Neither the '289 patent nor the '508 patent disclose or suggest the use of streptolysin O for the treatment of cancer.

### SUMMARY OF THE INVENTION

Herein described are means for treating connective tissue disorders by administering streptolysin O. Specifically, herein described are means for alleviating symptoms of a connective tissue disorder such as Dupuytren's contracture, scleroderma, Peyronie's disease, mastitis in animals, and claudication due to peripheral arterial disease by administering to a patient in need thereof, streptolysin O in an amount effective to treat one or more symptoms of the connective tissue disorder. As used herein, streptolysin O shall include the streptolysin O molecule as well as active fractions, analogs and derivatives thereof that maintain the biological activity of streptolysin O such as the ability to interact with the CD44 receptor or inhibit CD44 receptor mediated processes.

Herein described is the administration to a patient suffering from a connective tissue disorder such as tendonitis, Dupuytren's contracture, scleroderma, Peyronie's disease, mastitis in animals, and claudication due to peripheral arterial disease, an effective amount of streptolysin O. In one aspect, the tendonitis is equine tendonitis. A preferred route of administration is sublingually, but other routes including bucal, oral drench, anal, vaginal, nasal, intralesional, subcutaneous, intradermal, intramuscular, intrathecal, intravenous, inhalation or topical, by capsules, tablets, spray, topical lotions, creams, patches, or by intradermal or dermal punctures are contemplated.

Herein described is a pharmaceutical composition of streptolysin O and active fractions thereof for administering to a subject, or patient for alleviating symptoms of a connective tissue disorder such as tendonitis, Dupuytren's contracture, scleroderma, Peyronie's disease, claudication due to peripheral arterial disease, and mastitis wherein the streptolysin O is in an amount effective to treat one or more symptoms of said connective tissue disorder in combination with pharmaceutically-acceptable excipients. In one aspect, the tendonitis is equine tendonitis. Streptolysin O may be formulated in a number of phannaceutically-acceptable excipients including, but not limited to, water, saline, albumin, dextrose or any other pharmaceutically acceptable excipient known in the art. Means for alleviating symptoms of fibrotic conditions, such as but not limited to fibrosis of the kidney, liver, heart, lung, pancreas and other organs and further including reproductive fibrosis conditions such as uterine fibrosis and fallopian tube fibrosis are herein described. They comprise administering to a subject in need thereof, streptolysin O and active fractions thereof in an amount effective to treat one or more symptoms of the reproductive fibrosis including infertility and menstrual irregularities. The method may be applied to all types of mammalian subjects and in particular to equine and human subjects. Those of ordinary skill in the art can readily determine appropriate dosages for administration based on the therapy to be effected, the size of the subject and the mode of administration.

The streptolysin O may be formulated in a number of pharmaceutically-acceptable carriers or excipients including, but not limited to, water, saline, albumin, dextrose or any other pharmaceutically acceptable excipient known in the art. The precise dose will vary among patients and may readily be determined by those of ordinary skill in the art. In some embodiments, the streptolysin O is administered in a dosage amount ranging from about 0.0032 to 50 units (2 units/0.05 ml) per day and is preferably formulated in a liquid vehicle. In some embodiments, the streptolysin O is provided at a concentration of approximately 4 units as a single drop. A single drop of streptolysin O is within the range of 0.05 to 10 units. In some embodiments, a drop of streptolysin O is in the amount of 2 units as a single drop. In other embodiments, the streptolysin O is more administered in an amount ranging from about 0.01 to 10 units per day. In still other embodiments, the streptolysin O is administered in an amount ranging from about 0.1 to 8 units per day. In other embodiments, the administered dose of streptolysin O is from about 1 unit to about 5 units. In yet other embodiments, the administered dose of streptolysin O is about 2 units. A preferred route of human administration is sublingually, but other routes, such as bucal, oral drench, subcutaneous, intradermal, intramuscular, intrathecal, intravenous, inhalation or topical, are contemplated. For non-human animals such as horses, a preferred mode of administration is by subcutaneous administration at a dosage of 2 units per dose (0.2 cc).

Herein described are means for protecting subjects from the effects of neurotoxic agents and conditions comprising the step of administering neuroprotective amounts of streptolysin O. Neurotoxic agents are those capable of damaging or destroying neurons and include natural and synthetic agents present in the environment and further include natural metabolic products having neurotoxic properties. Neurotoxic conditions are not limited to those of a chemical origin and include damaging radiation and thermal conditions. In addition, the administration of streptolysin O may also be beneficial in repairing preexisting damage caused to neurons.

Herein described are methods for inhibiting CD44 receptor mediated processes comprising administering streptolysin O to cells expressing the CD44 receptor in amounts effective to inhibit said CD44 receptor mediated processes including, but not limited to, mobilization and orientation of hyaluronic acid generally and as involved in wound healing. Other CD44 mediated processes which may be susceptible to treatment with streptolysin O according to the invention include endometrial hyperplasia/carcinomas, orapharyngeal squamous cell carcinoma, breast carcinoma and panbronchiolitis. In particular, herein described are means of inhibiting hyaluronic acid binding to the CD44 receptor, by administering streptolysin O to cells expressing the CD44 receptor in an amount effective to inhibit said hyaluronic acid binding. The treatment may be carried out by administration of streptolysin O to subjects in which it is desired to inhibit CD44 receptor mediated processes including human subjects.

The present invention (claims 1-17) is based on the discovery that streptolysin O suppresses/inhibits the growth and/or invasion of a cancer cell that expresses a matrix metalloproteinase gene. In one aspect, described herein is the therapeutic inhibition of metastasis of cancer in a subject comprising administering streptolysin O to the subject in an amount effective to inhibit metastasis of the tumor. In one embodiment, the cancer is selected from the group consisting of glioma, melanoma, fibrosarcoma, and adenosarcoma. In another embodiment, the cancer is selected from the group consisting of human sarcomas and carcinomas. In yet another embodiment, the cancer is selected from the group consisting of myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, astrocytoma, medulloblastoma, craniopharyngioma, ependymoma, pinealoma, hemangioblastoma, acoustic neuroma, oligodendroglioma, meningioma. neuroblastoma, retinoblastoma; leukemias, e.g., acute lymphocytic leukemia and acute myelocytic leukemia (myeloblastic, promyelocytic, myelomonocytic. monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrboom's macroglobulinemia, and heavy chain disease.

In some embodiments, the treatment described herein further comprise administering a MMP inhibitor to said subject. Thus, also provided are means of inhibiting or suppressing metastasis of cancer in a subject comprising administering to said subject a combination therapy comprising streptolysin O and a MMP inhibitor wherein the combination therapy is administered in an amount effective to inhibit or suppress metastasis of cancer in the subject.

Also provided are means of inhibiting or suppressing metastasis of a cancer cell, the method comprising contacting the cancer cell with streptolysin O in an amount effective to inhibit or suppress metastasis of the cancer cell. In some embodiments, the cancer cell is from a cancerous tissue selected from the group consisting of glioma, melanoma, fibrosarcoma, and adenosarcoma. In some embodiments, the cancer cell is from a cancerous tissue selected from the group consisting of human sarcomas and carcinomas. In some embodiments, the cancer cell is from a cancerous tissue selected from the group consisting of myxosarcoma, liposarcoma, chondrosarcoma, osteogenic sarcoma, chordoma, angiosarcoma, endotheliosarcoma, lympllangiosarcoma, lymphangloendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, astrocytoma, medulloblastoma, craniopharyngioma, ependymoma, pinealoma, hemangioblastoma, acoustic neuroma, oligodendroglioma, meningioma, neuroblastoma, retinoblastoma; leukemias, e.g., acute lymphocytic leukemia and acute myelocytic leukemia (myeloblastic, promyelocytic, myelomonocytic. monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrboom's macroglobulinemia, and heavy chain disease.

Still other aspects involve combination therapy comprising the administration of streptolysin O to a subject in need thereof and standard of care therapeutics for the treatment of cancer. In the context of methods of the invention, "standard of care" refers to a treatment that is generally accepted by clinicians for a certain type of patient diagnosed with a type of illness. Exemplary standard of care regimens for the treatment of cancer include, but are not limited to, a standard of care chemotherapeutic, a standard of care radiotherapeutic, or a standard of care radiation regimen. For all varieties of cancers, for example, in one aspect is to improve standard of care therapy with co-therapy with streptolysin O described herein.

It should be understood, however, that the detailed description and the specific examples are given by way of illustration, and that the many various changes and modifications that will be apparent to those familiar with the field of the invention are also part of the invention.

Aspects of the invention described with the term "comprising" should be understood to include the elements explicitly listed, and optionally, additional elements. Aspects of the invention described with "a" or "an" should be understood to include "one or more" unless the context clearly requires a narrower meaning.

### BRIEF DESCRIPTION OF THE DRAWINGS

Figure 1 depicts the effects of streptolysin O on the contraction frequency in a nerve muscle co-culture in a glutamate intoxication model; and
Figure 2 depicts the effects of streptolysin O on the contraction frequency in a nerve muscle co-culture in a glutamate intoxication model.
Figure 3 shows the results of the cell invasion assay with MDA MB 231 cells after administration of streptolysin O at varying concentrations.
Figure 4 shows the results of the cell invasion assay with BT549 cells after administration of streptolysin O at varying concentrations.
Figure 5 shows the results of the cell invasion assay with MDA MB 231 cells after administration of recombinant streptolysin O at varying concentrations.

### DETAILED DESCRIPTION

Herein described are means for treating patients with symptoms of connective tissue disorders by any variety of modes of administration including, but not limited to, bucal, oral drench, anal, vaginal, nasal, intralesional, subcutaneous, intradermal, intramuscular, intrathecal, intravenous, inhalation or topical administration of a small amount of streptolysin O in a pharmaceutically acceptable excipient including water, saline, albumin, and dextrose. Internal organs with the potential for fibrotic conditions that can be treated by the methods of the invention include respiratory: lung, larynx, pharynx, nasal, sinisoids, Eustachian tubes, bronchioles (COPD, emphysema); gastric intestinal: intestinal adhesions, intestinal fibroids, "visceral", esophagus, liver, alimentary canal, hemorrhoids, rectal scarring, gall bladder ducts; circulatory and cardiac system heart, pericardia (pericardititis), ischemia, varicose veins, angina pectoralis, pancreas, lymph nodes; genital urinary kidneys, uterus and endometrium, polyps (vaginal and urethral), penis, vagina, fallopian tubes, urethra, bladder, prostate, ovaries; nervous: spinal cord, peripheral nerves, eyes, epidural and subdura (brain coverings), otic chambers, integument skin ulcers, scars, bums, acne cysts, other cysts, scleroderma; muscular/skeletal: ligaments and joints, skeletal muscle herein described are for treating Dupuytren's contracture including, but not limited to, treating the symptoms of thickening and contracture of the tissue beneath the skin of the palm of the hand and the limited everyday function with the hands.

Herein described are means for treating claudications due to peripheral arterial disease by administration of a small amount of streptolysin O. The means herein described are also useful for treating symptoms of peripheral arterial disease including, but not limited to, the intermittent claudication symptoms such as cramping, aching, numbness, lack of circulation, and/or pain of the extremities.

Herein described are means for treating patients with symptoms of Peyronie's Disease by administration of a small amount of streptolysin O. Methods of the invention are also useful for treating Peyronie's disease complications sufficient to treat symptoms of Peyronie's Disease including, but not limited to, painful erections or difficulty with intercourse.

Herein described are means for treating patients with symptoms of scleroderma by administration of a small amount of streptolysin O. The means herein described are also useful for treating scleroderma complications, including, but not limited to, Raynaud's Phenomenon, swelling of the hands and feet, pain and stiffness of the joints, thickening of the skin, joint contracture, digestive and gastrointestinal tract problems, Sjogen's Syndrome, facial and dental problems, kidney disease, heart disease, lung disease, extreme fatigue, generalized weakness, weight loss, vague aching of muscles, joints, and bones, and pulmonary hypertension.

Herein described are means for treating symptoms of chronic mastitis in bovines by administration of a small amount of streptolysin O.

The means herein described are also useful for treating mastitis complications characterized by redness, heat, pain, hardness or swelling accompanied by fever, a loss of appetite, and lower milk production of the bovine.

Herein described are means for treating symptoms of reproductive fibrosis including uterine and fallopian tube fibroses by administration of effective amounts of streptolysin O.

Herein described are means for treating tendonitis by administration of streptolysin O. In one aspect, the tendonitis is equine tendonitis. Methods of the invention are also useful for alleviating symptoms associated with equine tendonitis including, but not limited to, swelling, heat, and pain. If the tendonitis is more severe, the injury is usually accompanied by moderate lameness (2-3 on a scale of 5) with obvious swelling.

Herein are further provided neuroprotective means for prevention of the negative effects of neurotoxic agents on nerve cells by administration of effective amounts of streptolysin O.

Further, herein described are means for the intervention in CD44 receptor mediated conditions comprising the administration of streptolysin O to cells expressing the CD44 receptor in amounts effective to inhibit those processes. Also provided are methods of inhibiting hyaluronic acid binding to the CD44 receptor comprising administering streptolysin O to cells expressing the CD44 receptor in an amount effective to inhibit such hyaluronic acid binding to the CD44 receptor.

The present invention is based on the discovery that streptolysin O suppresses/inhibits the growth and/or invasion of a cancer cell that expresses a matrix metalloproteinase gene.

In one aspect, the invention provides a means of inhibiting or suppressing metastasis of a cancer cell, the method comprising contacting the cancer cell with streptolysin O in an amount effective to inhibit or suppress metastasis of the cancer cell.

The term "streptolysin O" as used with respect to the methods described herein means streptolysin O which has been modified by oxidation to eliminate cytotoxic effects while retaining important cholesterol binding characteristics on the cell membrane. Streptolysin O is readily oxidized in solution (Sigma Product Catalog).

As used herein "inhibition of MMP" includes inhibition of MMP activity, as well as inhibition of MMP production regardless of the mechanism of activity or production. This inhibition can be caused directly, e.g. by binding to MMP or its binding partner, by MMP inhibitors or MMP antibodies or by preventing it acting as a proteinase. The inhibition can also be caused indirectly, for example by inhibiting a pathway that results in MMP production. Inhibition causes a reduction in the MMP activity regardless of the exact mechanism of inhibition.

In another aspect, described herein are means of inhibiting or suppressing metastasis of a tumor in a subject comprising administering streptolysin O to the subject in an amount effective to inhibit or suppress metastasis of the tumor in the subject. In one embodiment, the tumor is a solid tumor.

In some embodiments, inhibiting or suppressing metastasis of the cancer cell (or tumor) comprises inhibiting the protein that contributes to metastasis of the cancer. In some embodiments, the protein is an enzyme that degrades extracellular matrix components and in some embodiments, the enzyme is a matrix metalloproteinase protein. For example, in some embodiments, the matrix metalloproteinase protein is selected from the group consisting of MMP-2, MMP-9, and MMP-2 and MMP-9.

In some embodiments, the cancer cell is a cell from a cancerous tissue selected from the group consisting of glioma, melanoma, fibrosarcoma, and adenosarcoma. In other embodiments, the cancer cell is from a cancerous tissue selected from the group consisting, but not limited to, human sarcomas and carcinomas, including but not limited to myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, astrocytoma, medulloblastoma, craniopharyngioma, ependymoma, pinealoma, hemangioblastoma, acoustic neuroma, oligodendroglioma, meningioma, neuroblastoma, retinoblastoma; leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrboom's macroglobuhnemia, and heavy chain disease.

The subjects treated are desirably human subjects, although it is to be understood that the principles of the presently disclosed subject matter indicate that the presently disclosed subject matter is effective with respect to invertebrate and to all vertebrate species, including mammals, which are intended to be included in the term "subject". Moreover, a mammal is understood to include any mammalian species in which treatment or prevention of cancer is desirable, particularly agricultural and domestic mammalian species.

In some embodiments, streptolysin O is administered 1, 2, 3, 4, 5, 6, 7, 8, 9, 10 or more times daily for a period of 1, 2, 3, 4, 5, 6 or more weeks. Additional therapy may be administered on a period basis, for example, daily, weekly or monthly.

The dose of streptolysin O administered to the subject can be determined by the physician, taking into account, age, sex, weight, etc. of the subject. In some embodiments, the administered dose of streptolysin O is at least 0.01, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3.0, 4.0, 5.0, 6.0, 7.0, 8.0, 9.0, 10.0 or more Intetnational units. In one embodiment, the administered dose of streptolysin O is 2 units.

### Combination Therapy

The therapeutic means described herein further comprise administering a MMP-2 and/or MMP-9 inhibitor to the subject. As used herein, "MMP inhibitor" is an agent that directly or indirectly inhibits MMP activity. This includes an agent that blocks MMP activity or an agent that blocks a pathway of MMP production. The agent causes a reduction in MMP activity in a cancer cell (or cancerous tissue) regardless of the mechanism of its action. Representative examples of MMP Inhibitors include Tissue Inhibitors of Metalloproteinases (TIMPs) (e.g., TIMP-1, TIMP-2, TIMP-3, or TIMP-4), O₂-macroglobulin, tetracyclines (e.g., tetracycline, minocycline, and doxycycline), hydroxamates (e.g., BATIMASTAT, MARIMISTAT and TROCADE), chelators (e.g., EDTA, cysteine, acetylcysteine, D-penicillamine, and gold salts), synthetic MMP fragments, succinyl mercaptopurines, phosphonamidates, and hydroxaminic acids.

Broad-spectrum inhibitors that inhibit more than one type of MMP, such as are also contemplated. Exemplary broad spectrum MMP inhibitors include, but are not limited to, GM6001, batimastat, marimastat, prinomastat, BAY 12-9566, MMI270(B), BMS-275291, and metastat. Inhibitors that are capable of inhibiting MMP2, MMP9 or both MMP2 and MMP9 are specifically contemplated. Exemplary MMP-2/MMP-9 inhibitors includes, but is not limited to, SB-3CT. For example, in one embodiment, combination therapy comprising the administration of streptolysin O and a MMP-2/MMP-9 inhibitor is specifically contemplated.

Assays for measuring MMP inhibition/suppression are readily known in the art, and include, for example, the following: Cawston T. E., Barrett A. J., "A rapid and reproducible assay for collagenase using [14C] acetylated collagen," Anal. Biochem. 35:1961-1965 (1963); Cawston T. E., Murphy G. "Mammalian collagenases," Methods in Enzymology 80:711 (1981); Koshy P. T. J., Rowan A. D., Life P. F., Cawston T. E., "96-well plate assays for measuring collagenase activity using (3)H-acetylated collagen," Anal. Biochem. 99:340-345 (1979); Stack M. S., Gray R. D., "Comparison of vertebrate collagenase and gelatinase using a new fluorogenic substrate peptide," J. Biol. Chem. 264:4277-4281 (1989); and Knight C. G, Willenbrock F., Murphy G, "A novel coumarin-labelled peptide for sensitive continuous assays of the matrix metalloproteinases," FEBS Lett 296:263-266 (1992).

In some aspects, the therapeutic means described herein further comprise administering a standard of care cancer therapy to the subject. "Standard of care" refers to a treatment that is generally accepted by clinicians for a certain type of patient diagnosed with a type of illness. Exemplary standard of care anti-cancer agents include, but are not limited to, a standard of care chemotherapeutic, a standard of care radiotherapeutic, or a standard of care radiation regimen. For all varieties of cancers a, for example, one aspect is to improve standard of care therapy with co-therapy with the streptolysin O described herein.

Examples of suitable chemotherapeutic and radiotherapeutic agents include, but are not limited to: an anti-metabolite; a DNA-damaging agent; a cytokine useful as a chemotherapeutic agent; a covalent DNA-binding drug; a topoisomerase inhibitor; an anti-mitotic agent; an anti-tumor antibiotic; a differentiation agent; an alkylating agent; a methylating agent; a hormone or hormone antagonist; a nitrogen mustard; a radiosensitizer; a photosensitizer; a radiation source, optionally together with a radiosensitizer or photosensitizer; or other commonly used therapeutic agents. Specific examples of chemotherapeutic agents useful in methods of the present invention are listed in Table 1 below.

| | **TABLE 1** | |
|---|---|---|
| **Alkylating agents** | | |
| Nitrogen mustards | Epioodophylotoxins | **Hormones and antagonist** |
| mechlorethamine | etoposide | Adrenocorticosteroids/ antagonists |
| cyclophosphamide | teniposide | prednisone and equivalents |
| ifosfamide | Antibiotics | dexamethasone |
| melphalan | actimomycin D | ainoglutethimide |
| chlorambucil | daunomycin (rubido-mycin) | Progestins |
| Nitrosoureas | doxorubicin (adria-mycin) | hydroxyprogesterone caproate |
| carmustine (BCNU) | mitoxantroneidarubicin | medroxyprogesterone acetate |
| lomustine (CCNU) | bleomycinsplicamycin (mithramycin) | megestrol acetate |
| semustine (methyl-CCNU) | | Estrogens |
| Ethylenimine/Methyl-melamine | mitomycinC | diethylstilbestrol |
| thriethylenemelamine (TEM) | dactinomycin | ethynyl estradiol/ equivalents |
| triethylene thiophosphoramide (thiotepa) | Enzymes | Antiestrogen |
| | L-asparaginase | tamoxifen |
| hexamethylmelamine (HMM, altretamine) | **Biological response modifiers** | Androgens |
| | interferon-alpha | testosterone propionate |
| Alkyl sulfonates | IL-2 | fluoxymesterone/equivalents |
| busulfan | G-CSF | Antiandrogens |
| Triazines | GM-CSF | flutamide |
| dacarbazine (DTIC) | **Differentiation Agents** | gonadotropin-releasing |
| Antimetabolites | retinoic acid derivatives | hormone analogs |
| Folic Acid analogs | **Radiosensitizers** | leuprolide |
| methotrexate | metronidazole | Nonsteroidal antiandrogens |
| Trimetrexate | misonidazole | flutamide |
| Pemetrexed | desmethylmisonidazole | **Photosensitizers** |
| Multi-targeted antifolate | pimonidazole | hematoporphyrin derivatives |
| Pyrimidine analogs | etanidazole | Photofrin®) |
| 5-fluorouracil | nimorazole | benzoporphyrin derivatives |
| fluorodeoxyuridine | RSU 1069 | Npe6 |
| gemcitabine | EO9 | tin etioporphyrin (SnET2) |
| cytosine arabinoside (AraC, cytarabine) | RB 6145 | pheoboride-a |
| | SR4233 | bacteriochlorophyll-a |
| 5-azacytidine | nicotinamide | naphthalocyanines |
| 2,2'-difluorodeoxy-cytidine | 5-bromodeozyuridine | phthalocyanines |
| Purine analogs | 5-iododeoxyuridine | zinc phthalocyanines |
| 6-mercaptopurine | bromodeoxycytidine | |
| 6-thioguanine | **Miscellaneous agents** | |
| azathioprine | Platinium coordination complexes | |
| 2'-deoxycoformycin (pentostatin) | cisplatin | |
| | Carboplatin | |
| erythrohydroxynonyl-adenine | oxatiplatin | |
| (EHNA) | Anthracenedione | |
| fludarabine phosphate | mitoxantrone | |
| 2-chlorodeoxyadenosine | Substituted urea | |
| (cladribine, 2-CdA). | hydroxyurea | |
| **Type I Topoisomerase Inhibitors** | Methylhydrazine derivatives | |
| camptothecin | N-methylhydrazine (MIH) | |
| topotecan | procarbazine | |
| irinotecan | Adrenocortical suppressant | |
| **Natural products** | mitotane (*o*,*p*'- DDD) | |
| Antimitotic drugs | ainoglutethimide | |
| paclitaxel | Cytokines | |
| Vinca alkaloids | interferon (*, *, *) | |
| vinblastine (VLB) | interleukin-2 | |
| vincristine | | |
| vinorelbine | | |
| Taxotere® (docetaxel) | | |
| estramustine | | |
| stramustine phosphate | | |

Cytokines that are effective in inhibiting tumor metastasis are also contemplated for use in the combination therapy. Such cytokines, lymphokines, or other hematopoietic factors include, but are not limited to, M-CSF, GM-CSF, TNF, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IFN, TNFα, TNF1, TNF2, G-CSF, Meg-CSF, GM-CSF, thrombopoietin, stem cell factor, and erythropoietin.

In some examples, the method also includes monitoring the effect of treatment on the tumor. For example, the size of the tumor can be determined. The therapeutic described herein and/or standard of care therapy would be provided in a combined amount effective to kill or inhibit proliferation of the cancer by killing and/or inhibiting the proliferation of the cancer cells and/or the endothelia of blood and lymphatic vessels supplying and serving the cancer cells. This process may involve contacting the cells with streptolysin O as described herein and one or more standard of care therapies at the same time. This may be achieved by contacting the cell with a single composition or pharmacological formulation that includes both agents, or by contacting the cell with two distinct compositions or formulations, at the same time, wherein one composition includes the streptolysin O and the other includes a standard of care therapy.

Alternatively, the therapeutic treatment employing streptolysin O as described herein may precede or follow the standard of care therapy by intervals ranging from minutes to weeks. In embodiments where the standard of care therapy and streptolysin O are administered separately, one would generally ensure that a significant period of time did not expire between the times of each delivery, such that the standard of care therapy and the streptolysin O would still be able to exert an advantageously combined effect. In such instances, it is contemplated that one would administer both modalities within about 12-24 hours of each other. In some embodiments, the modalities would be administered within 6-12 hours of each other. In other embodiments, the modalities are administered within 1-6 hours of each other. Repeated treatments with one or both agents are specifically contemplated.

In some embodiments, streptolysin (either alone or in combination with an MMP inhibitor or standard of care therapeutic as described herein) is delivered directly to the subject (or cancer cell) or in compositions along with suitable carriers or excipients, as is well known in the art.

The following Examples illustrate the treatment of psychological conditions, and, in particular, with respect to means of treating connective tissue disorders. In particular, streptolysin O was used to treat these various psychological disorders. The streptolysin O was purchased from Sigma Chemicals, St. Louis, MO. Numerous improvements and further aspects of the invention are apparent to the skilled artisan upon consideration of the Examples, which follows.

Examples 1-8 illustrate the treatment of connective tissue disorders, including example 8 which illustrates a method of treating bovine mastitis. Examples 9 and 10 illustrate the administration of streptolysin O to subjects suffering from reproductive fibrosis. Examples 11 and 12 illustrate means of protecting nerve cells from the effects of neurotoxic agents. Example 13 shows the results of competitive inhibition assays carried out between hyaluronic acid and streptolysin O on human keratinocyte CD44 receptors. Examples 14-16 illustrate the administration of streptolysin O to subjects suffering from tendonitis. Example 17 demonstrates that streptolysin O inhibits/suppresses cancer metastases in two metastastic cancer cell lines (MBA MB231 and BT549 cells).

### EXAMPLE 1 (not part of the invention)

An 85-year old female patient suffered from calf pain due to peripheral arterial disease. She began treatment with one drop (2 units/0.05 ml) of streptolysin O two to four times daily by sublingual administration. Before treatment, she could only walk three minutes without experiencing pain in her calf. With treatment, she has been able to walk nine minutes without pain in her calf.

### EXAMPLE 2 (not part of the invention)

An 80-year old female patient suffered from lower extremity pain due to peripheral arterial disease on her right side. She began a treatment regimen of one drop (2 units/0.05 ml) of streptolysin O four times daily by sublingual administration. With this treatment regimen, her pain has been relieved.

### EXAMPLE 3 (not part of the invention)

A 72-year old male was diagnosed with lower leg pain due to peripheral arterial disease by his physician. He began treatment with streptolysin O at a rate and amount of one (2 units/0.05 ml) drop, four times daily by sublingual administration. The treatment regimen has significantly decreased the leg pain and further improved his energy, ability to work, and improved his overall quality of life.

### EXAMPLE 4 (not part of the invention)

A 67-year old male was diagnosed with Dupuytren's contracture in one hand. He began treatment with streptolysin O at a rate and amount of one (2 units/0.05 ml) drop, four times daily by sublingual administration. After 14 days of treatment, the symptoms of the disease progressively reversed with each new treatment.

### EXAMPLE 5 (not part of the invention)

A 64-year old male was diagnosed with Dupuytren's contracture in both hands by his physician. He began treatment with streptolysin O at a rate and amount of one (2 units/0.05 ml) drop, four times daily by sublingual administration. After 14 days of treatment, the symptoms of the disease progressively reversed in both hands with each new treatment.

### EXAMPLE 6 (not part of the invention)

A 57-year old male patient was diagnosed with Peyronie's disease by his physician. He began treatment with streptolysin O at a rate and amount of one (2 units/0.05 ml) drop, three times daily by sublingual administration. After 30 days of treatment, improvements in the contracture of his penis were noted by the patient and his physician.

### EXAMPLE 7 (not part of the invention)

A male patient was diagnosed with Peyronie's disease by his physician. He began treatment with streptolysin O at a rate and amount of one (2 units/0.05 ml) drop, three times daily by sublingual administration. After 30 days of treatment, improvements in the contracture of his penis were noted by the patient and his physician.

### EXAMPLE 8 (not part of the invention)

Bovines, which have lost one or more quarters to mastitis, were treated using one dose of streptolysin O (2 units/0.05 ml) drop, twice daily for thirty days by subcutaneous administration. The quarters damaged due to mastitis were reclaimed and produced milk after treatment.

### EXAMPLE 9 (not part of the invention)

According to this example, streptolysin O was administered to a 27 year old human female with a history of failure to conceive. The subject was diagnosed as suffering with fibroid scarring on the uterus and fallopian tubes and was assessed as having less than a 20% chance of becoming pregnant. Specifically, streptolysin O was administered at the rate of one (2 units/0.05ml) drop, four times daily by sublingual administration. The subject became pregnant within six weeks of the initiation of therapy and successfully delivered a healthy child.

### EXAMPLE 10 (not part of the invention)

According to this example, streptolysin O was administered to ten mares diagnosed as suffering from periglandular or uterine fibrosis. Mares can become infertile after several pregnancies as a consequence of periglandular or uterine fibrosis which prevents implantation and/or dilation of the uterus such as to be insufficient to accommodate the growing fetus.

Ten mares diagnosed as suffering from periglandular or uterine fibrosis were treated by subcutaneous administration of streptolysin O at a dosage of 2 units per dose (0.2 etc). The horses were treated by streptolysin O administration twice daily for two weeks followed by one dose daily for one week. The results of histopathological evaluation of the first three horses by endometrial biopsies both before and after treatment are set out in Table I below. Such endometrial biopsies are graded as category I (essentially normal) through categories IIA, IIB, and III with category III being the worst.

| TABLE I | | |
|---|---|---|
| Horse No. | Initial Evaluation | Evaluation 60 Days Post Treatment |
| 1 | IIB-III | IIB |
| 2 | III | IIB |
| 3 | III | IIA |

The first three horses were bred after administration of the streptolysin O and two of the horses were safely in foal (having been carrying a live fetus more than 50 days). The fourth horse was not bred but was also found to have reduced uterine fibrosis as determined by endometrial biopsy. Of the next six horses no histopathological evaluation was available but three had conceived after treatment according to the invention.

### EXAMPLE 11 (not part of the invention)

According to this example, the neuroprotective effects of streptolysin O were determined in a glutamate intoxication model utilizing the nerve nerve-muscle co-culture developed by Askanas and Engel, Neurology 25:58-67 (1975). This culture makes it possible to create striated human muscle fiber innervations with rat spinal chord explants and dorsal root ganglions. After 15 days of co-culture, the muscle fibers show spontaneous contractile activity. In this co-culture, it is then possible to induce cell death by intoxication using neurotoxic agents such as glutamate or hydrogen peroxide. Such intoxications are time dependent and the viability and functionality of the motoneurons can be studied from two parameters: muscle contractile activity and apoptosis induction, quantified by an ELISA method on the basis of quantification of cytoplasmic histone-associated DNA fragments.

Glutamate is the main excitatory neurotransmitter in the mammalian CNS, but over-stimulation of its receptors causes neuro-degeneration. According to empirical protocol, in nerve-muscle co-cultures, 10 mM of glutamate in the medium decreases contractile activity after one day and cause motoneuron cell death by apoptosis after 1 week. This motoneuron cell death induces muscle fiber degeneration.

According to this example, streptolysin O at two concentrations, 0.01% and 1% (four wells per condition) was incubated for 9 days in an established nerve-muscle co-culture. The conditioned culture media was replaced each day. Cultures were then intoxicated with either glutamate 10 mM or hydrogen peroxide (H₂O₂) 800µM and the neurotoxic effects were evaluated by (1) quantification of the frequency of contractions and (2) by cell death level as measured by quantification of apoptosis.

The contraction frequency of the cells was then measured and recorded by image analysis software respectively before incubation, after 1 hour, 24 hours, 48 hours and 72 hours of intoxication with or without incubation with streptolysin O 0.01% and 1% on their own. The results are given as a percentage of the contraction frequency compared to the contraction frequency before incubation expressed as 100%. Two co-incubations were tested, glutamate 10mM and H₂O₂ 800(M with the product SO 0.01% and 1% (4 wells per condition). These results show that streptolysin O was responsible for a dramatic increase of the contraction frequency reaching a plateau after 24 hours, 48 hours and 72 hours.

After 24 hours incubation with a 10mM concentration of glutamate an increase in the contraction frequency of the selected muscle fibers was observed. After 48 hours incubation with a 10mM glutamate concentration the contraction frequency of the muscle fiber decreased to reach a lower level than its basic level. The contraction frequency after 10mM glutamate intoxication in combination with streptolysin O incubation increased during the first 24 hours and after 48 hours remained higher than glutamate intoxication on its own.

The effect of streptolysin O on the contraction frequency of a muscle fiber (4 wells per condition) selected from a nerve-muscle co-culture after 1 hour, 24 hours, 48 hours and 72 hours of intoxication with 10mM L-glutamate. Specifically, the data are shown at two concentrations (0.01% and 1%) compared to a normalized control response before incubation expressed as 100%.

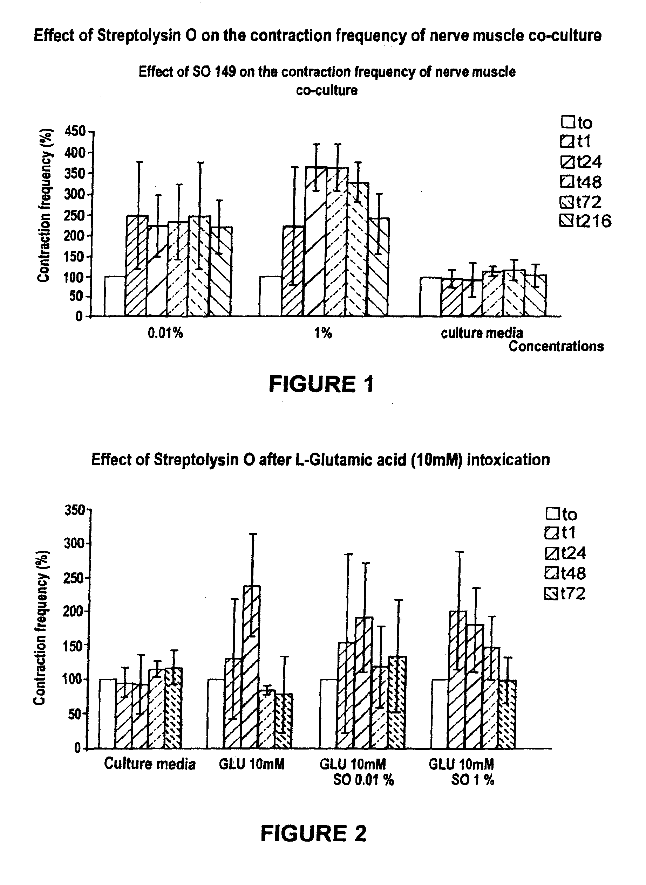

The results shown in Figure 1 illustrates that the contraction frequency, with streptolysin O at 0.01%, dramatically increased to reach a plateau of almost 250% of the control response and that at a streptolysin O concentration of 1% increase the contraction frequency even greater.

The effect of SO at two concentrations (0.0 1 % and 1%) on the contraction frequency of nerve muscle co-cultures intoxicated with 10mM L-glutamate compared to the normalized control response before incubation expressed as 100 % (4 fibers analysed per condition) is shown in Fig. 2. Fig. 2 illustrates that the contraction frequency after 1 hour and 24 hours of incubation with 10mM L-glutamate dramatically increased and shows wide standard deviations. After 48 hours incubation, the contraction frequency of the muscle fiber decreased to recover its basic level (100%).

After a 1-hour incubation period, the effect ofstreptolysin O at 0.01% after 10mM L-Glutamate intoxication showed an increase to almost 150% of the control response and was almost to 200% after 24 hours. After 48 and 72 hours, the contraction frequency still reached more than 120% of the control response. After a 1-hour incubation period in presence of 10 mM glutamate, SO 149 1% lead to an increase to almost 200% of the control response and was almost the same after 24 hours. After 48 hours, the contraction frequency still reached 150% and it recovered the basic level (100% of the control response) after 72 hours.

These results show that after 10mM Glutamate intoxication, streptolysin O has both a myostimulating effect and a neuroprotective effect analyzed by a cell apoptosis quantification.

### EXAMPLE 12 (not part of the invention)

According to this example, the neuroprotective effects of streptolysin O were analyzed by quantification of cell apoptosis after 9 days of hydrogen peroxide intoxication (800µM) and Glutamate (10mM L-glutamate) intoxication. Negative and positive apoptosis controls were respectively (a) culture media, (b) glutamate 10mM and (c) H₂O₂ 800µM and apoptosis was evaluated using a Cell Death Detection ELISA kit" (Roche).

Specifically, the occurrence of apoptosis of nerve muscle co-cultures was measured using Optic Density at 405 nm after 9 days of (a) incubation with streptolysin O at two concentrations, 0.01% and 1% (b) intoxication with 10mM L-glutamate and 800µM hydrogen peroxide and (c) incubation with both L-glutamate 10mM and hydrogen peroxide 800µM in combination with streptolysin O at two concentrations 0.01% and 1%.

The results showed that the level of cell death from apoptosis within the culture with streptolysin O 0.01% was approximately the same as the control culture (0.3 OD). The glutamate intoxications showed a cell death level from apoptosis which was approximately the same as the control culture. The cell death level from apoptosis within the culture with both the glutamate and SO 0.01% or 1% incubation was similar to the control culture. The hydrogen peroxide intoxication showed that cell death level is slightly higher than the control culture. The cell death level with both the hydrogen peroxide and streptolysin O at concentrations of 0.01% or 1% incubation was similar to the culture intoxicated with hydrogen peroxide. These results suggest that the neuroprotective effects of streptolysin O are not related to an anti-apoptotic effect.

### EXAMPLE 13 (not part of the invention)

According to this Example, a competitive inhibition assay was carried out between the hyaluronic acid and streptolysin O on human keratinocyte CD44 receptors. Streptolysin O incubated in the medium of five different keratinocyte and melanocyte human primary cultures did not induce cytotoxicity. However in the same culture conditions, streptolysin O at both 1X and 0.1X concentrations induced an increase of keratinocyte cell growth but not melanocyte cell growth.

The evaluation of incidence of streptolysin O on cell surface markers of keratinocytes using microscopy analysis was determined by an immunofluorescence labeling carried out with monoclonal antibodies. Streptolysin O was found to have an influence on five keratinocyte markers (CD44, CD47, CD40, CD49-c and MMP-2). In particular, streptolysin O at 1X, 0.1X and 0.01X appeared to maintain expression of CD44 in keratinocytes after 26 hours of culture compared to loss of staining with placebo.

It has also been observed that CD44 was the hyaluronic acid receptor (HA). In order to determine the effects of streptolysin O in the CD44 - HA function, a competitive inhibition assay was carried out between hyaluronic acid (HA) and streptolysin O on the keratinocyte CD44 receptor. Specifically, a competitive inhibition between hyaluronic acid (HA) and streptolysin O (MIL001) on the keratinocyte CD44 receptor was tested on 01-010 cell culture (15 000 keratinocytes / well). This culture were treated with at (1) streptolysin O concentration (0.01X) and at six (6) different hyaluronic acid HA concentrations (0%, 0.0001%, 0.0005%, 0.001%, 0.005% and 0.01%) and at 3 different incubation times (1, 20, and 44 hours). The inhibition was measured by an immunofluorescence labeling performed with the anti Human CD44 monoclonal antibody using microscopic analysis.

Specifically, normal human keratinocytes were thawed and seeded with KGM medium (Bio Whittaker, MD) during the first passage. At subconfluence, keratinocytes were trypsinized, enumerated and cultured with KGM medium in flat-bottomed microtiter plates with 15 000 keratinocytes per well. The cultures were incubated at 37°C in a humidified 5% CO₂ atmosphere.

Streptolysin O 100X (200 units) was diluted at 0.01X. was added or not (control placebo) in 96 well microplates 24 hours after the cell seeding and maintained in culture for 1 hour, 20 hours and 44 hours. Different concentrations of hyaluronic acid solution at 0.1%, 0.05%, 0.01%, 0.005% and 0.001% were diluted at 1/10 in the different culture wells and added to the wells. At the end of each incubation time (1 hour, 20 hours and 44 hours), the keratinocytes were washed and fixed 5 minutes with ethanol/acetic acid (95/5) at -20°C. Cells were then extensively washed with buffer solution and were stored at 4°C until staining with monoclonal antibodies to human CD44 antigen phycoerythrin labeled (Caltag Laboratories).

A quantitative results analysis was then carried out on each culture. This quantitative analysis gives the tendency of the CD44 immunostaining with placebo or MIL001 0.01X as a function of HA concentration. The results show that for placebo samples CD44 staining is increased at the 2 higher HA concentrations 0.001% and 0.01%; however at 0.005% the staining is similar to lower HA concentrations.

The results after 1 hour and after 20 hours of culture showed no statistically significant difference between streptolysin O and placebo with respect to inhibiting hyaluronic acid binding to the CD44 receptor.

The results after 44 hours of culture show that CD44 expression is stronger with MIL001 0.001X in the presence of 0% hyaluronic acid than with placebo and confirms that streptolysin O increases CD44 expression on keratinocytes. CD44 staining is stronger with the mixture of MIL001 10.001X with 0.0001% hyaluronic acid but decreases with other concentrations of hyaluronic acid back to levels observed with placebo. Thus, hyaluronic acid inhibits the effect of streptolysin O at MIL001 0.001X which has been demonstrated to increase CD44 receptor expression compared to placebo.

The results after 44 hours of competition also indicate that lower concentrations of hyaluronic acid (0.0001% and 0.0005%) increase CD 44 receptor expression compared to the absence of hyaluronic acid. Then the expression of CD 44 decreases at higher HA concentrations yet is still higher than with the negative control (placebo + HA 0%).

Further, in samples incubated with streptolysin O at MIL001 0.001X CD44 receptor expression is constant compared to results obtained in the absence of hyaluronic acid whatever hyaluronic acid concentration is present in the culture. In other words, hyaluronic acid has no effect on CD44 receptor expression on keratinocytes in culture in the presence of streptolysin O at MIL001 0.001X.

Moreover, the results show that HA inhibits the increased expression of CD44 receptor on keratinocytes induced by MIL001 0.01X incubation and thereby shows competition between streptolysin O (MIL001) and hyaluronic acid (HA) for CD44 receptor binding after 44 hours of culture.

### EXAMPLE 14 (not part of the invention)

A 3-year old thoroughbred gelding suffered an acute tear of the medial branch of the right front suspensory ligament. After being treated for 30 days with streptolysin-O (0.2 cc streptolysin O twice daily (2 Units per dose) by subcutaneous administration) the attending veterinarian reported "significant resolution" of the medial branch.

### EXAMPLE 15 (not part of the invention)

A 3-year old thoroughbred colt had a small tear in the superficial digital flexor tendon. After being treated for 30 days with streptolysin-O (0.2 cc streptolysin O twice daily (2 Units per dose) by subcutaneous administration), the tear had significantly improved and the horse was being hand-walked daily.

### EXAMPLE 16 (not part of the invention)

A 2-year old Standardbred filly, a trotter used competitively, presented with a lame right front leg graded by the attending veterinarian as being a three out of a possible five with respect to severity. There was moderate effusion and pain associated with the lateral suspensory branch. A core lesion on the suspensory branch was detected by ultrasound. The filly was treated with streptolysin-O (0.2 cc streptolysin O twice daily (2 Units per dose) by subcutaneous administration for thirty days and then once daily thereafter) combined with stall rest for sixty days. At sixty days, there was no pain or effusion, the lesion size was significantly reduced, and the filly was sound when trotting, thus able to return to normal training.

### EXAMPLE 17

The effect of streptolysin O on malignant metastatic cancer cell lines was determined by the Matrigel Invasion Cell Assay as described in Rapesh, L., Invasion Metastasis, 9:192-208, 1989, the disclosure of which is incorporated herein by reference in its entirety. The assay was used to measure the reduction of cancer cell migration due to the incubation of cells with streptolysin O.

Cancer cells (either MDA MB 231 malignant metastatic breast cancer cells or BT549 malignant metastatic breast cancer cells) were added to the Matrigel (BD Biosciences) at concentrations of 100,000; 150,000 or 250,000 cells per plate for 90 minutes prior to addition of either streptolysin O (Sigma-Aldrich) or recombinant streptolysin O (rSLO, Capricorn Corporation LLC), which was diluted in deionized water to various concentrations (0.2, 2,5, 10) and 20 International Units per plate) which thereby oxidized streptolysin O to eliminate its cytotoxic effects. The cells were allowed to migrate into and through the gel towards the "attractant" (10% Fetal Bovine Serum) for 22 hours at 37°C. Migrating cells were captured on a filter at the end of the gel, stained with crystal violet and counted. Data were plotted as cells per square area vs. concentration of streptolysin (in which cells were incubated).

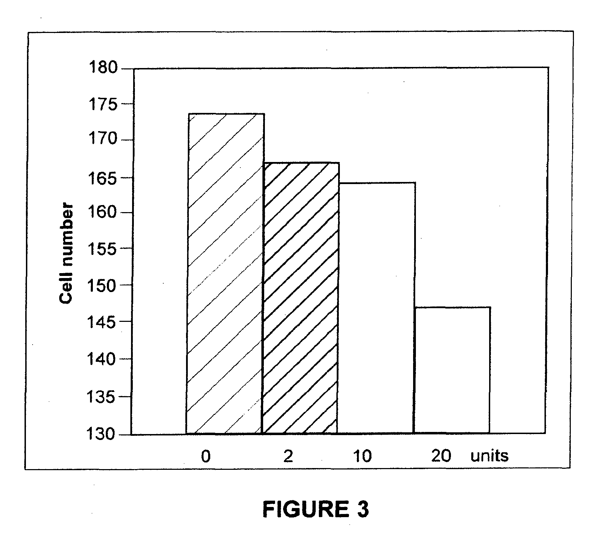

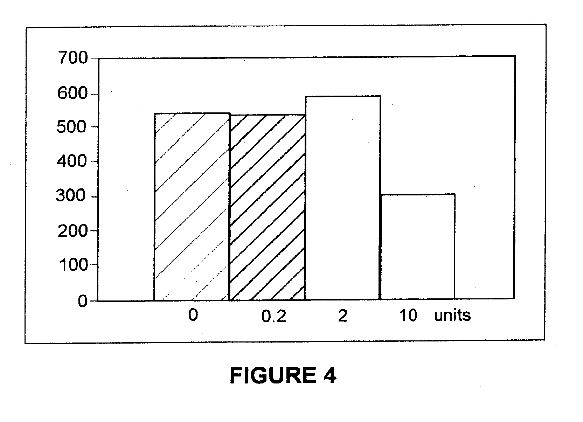

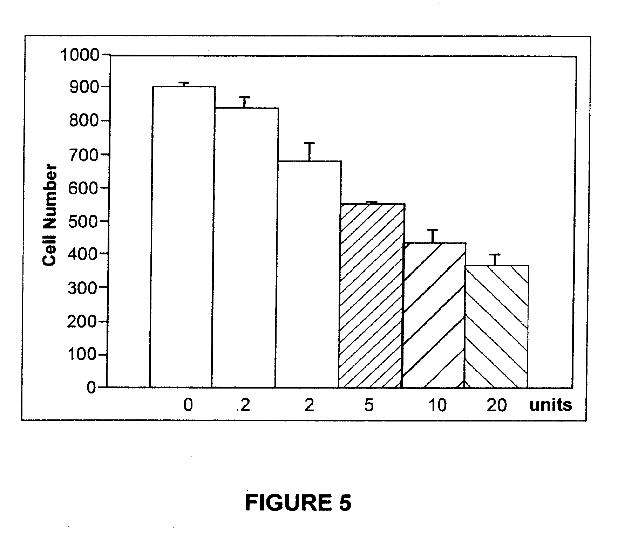

The experimental results show a measurable effect on cell migration by incubating the metastatic cancer cells with both streptolysin O and rSLO. The reduction in cell number reflects fewer cells migrating to the filter, which could be due to a reduction in the number of cells migrating or a slowing of the rate of migration. In either case the effect is a reduction in the number of cancer cells vs control (no streptolysin O) and it is dose dependent. For example, Figure 3 shows that all concentrations of streptolysin O assayed (2, 10 and 20 units) demonstrated a reduction of cell migration in MDA MB 231 cells, with 20 units demonstrating the greatest inhibitory effect. Similarly, Figure 5 shows that all concentrations of rSLO assayed (0.2, 2, 5, 10 and 20 units) reduced cell migration of MDA MD 231 cells, with 20 units demonstrating the greatest inhibitory effect. Figure 4 shows that 10 units streptolysin O reduced cell migration of BT549 cells.

Numerous modifications and variations in the practice of the invention are expected to occur to those of skill in the art upon consideration of the presently preferred embodiments thereof. Consequently, the only limitations which should be placed upon the scope of the invention are those which appear in the appended claims.

## Claims

1. Streptolysin O (SLO) for use in inhibiting metastasis of cancer in a subject comprising administering streptolysin O (SLO) to the subject in an amount effective to inhibit metastasis of the tumor.

2. Streptolysin O (SLO) for use according to claim 1, wherein the cancer is selected from the group consisting of glioma, melanoma, fibrosarcoma, and adenosarcoma.

3. Streptolysin O (SLO) for use according to claim 1, wherein the cancer is selected from the group consisting of human sarcomas and carcinomas.

4. Streptolysin O (SLO) for use according to claim 3, wherein the cancer is selected from the group consisting of myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, astrocytoma, medulloblastoma, craniopharyngioma, ependymoma, pinealoma, hemangioblastoma, acoustic neuroma, oligodendroglioma, meningioma, neuroblastoma, retinoblastoma; leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrboom's macroglobulinemia, and heavy chain disease.

5. Streptolysin O (SLO) for use according to claim 1, wherein the SLO is administered to the subject by a route selected from the group consisting of intramuscular, sublingual, intravenous, subcutaneous, and intrathecal.

6. Streptolysin O (SLO) for use according to claim 5, wherein the SLO is administered sublingually.

7. Streptolysin O (SLO) for use according to claim 5, wherein the SLO is administered subcutaneously.

8. Streptolysin O (SLO) for use according to claim 1, wherein the SLO is administered at dose from about 0.01 units to about 10 units.

9. Streptolysin O (SLO) for use according to claim 8, wherein the SLO is administered at dose from about 1 unit to about 5 units.

10. Streptolysin O (SLO) for use according to claim 9, wherein the SLO is administered at dose of about 2 units.

11. Streptolysin O (SLO) for use according to claim 1, wherein the SLO is administered at least twice daily.

12. Streptolysin O (SLO) for use according to claim 1, further comprising administering a MMP inhibitor to said subject.

13. Steptolysin O (SLO) in combination with an MMP inhibitor for for use in inhibiting or suppressing metastasis of cancer in a subject comprising administering to said subject a combination therapy comprising
(a) steptolysin O (SLO) and
(b) a MMP inhibitor
wherein the combination therapy is administered in an amount effective to inhibit or suppress metastasis of cancer in the subject.

14. Streptolysin O (SLO) for use in inhibiting or suppressing metastasis of a cancer cell by contacting the cancer cell with streptolysin O (SLO) in an amount effective to inhibit or suppress metastasis of the cancer cell.

15. Streptolysin O (SLO) for use according to claim 14, wherein the cancer cell is from a cancerous tissue selected from the group consisting of glioma, melanoma, fibrosarcoma, and adenosarcoma.

16. Streptolysin O (SLO) for use according to claim 15, wherein the cancer cell is from a cancerous tissue selected from the group consisting of human sarcomas and carcinomas. -

17. Streptolysin O (SLO) for use according to claim 16, wherein the cancer cell is from a cancerous tissue selected from the group consisting of myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, astrocytoma, medulloblastoma, craniopharyngioma, ependymoma, pinealoma, hemangioblastoma, acoustic neuroma, oligodendroglioma, meningioma, neuroblastoma, retinoblastoma; leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrboom's macroglobulinemia, and heavy chain disease.

## Patentansprüche

1. Streptolysin O (SLO) zur Verwendung beim Hemmen von Krebsmetastasen bei einem Subjekt, beinhaltend das Verabreichen von Streptolysin O (SLO) an das Subjekt in einer Menge, die wirksam ist, um Metastasen des Tumors zu hemmen.

2. Streptolysin O (SLO) zur Verwendung gemäß Anspruch 1, wobei der Krebs aus der Gruppe ausgewählt ist, die Gliom, Melanom, Fibrosarkom und Adenosarkom umfasst.

3. Streptolysin O (SLO) zur Verwendung gemäß Anspruch 1, wobei der Krebs aus der Gruppe ausgewählt ist, die menschliche Sarkome und Karzinome umfasst.

4. Streptolysin O (SLO) zur Verwendung gemäß Anspruch 3, wobei der Krebs aus der Gruppe ausgewählt ist, die umfasst: Myxosarkom, Liposarkom, Chondrosarkom, osteogenes Sarkom, Chordom, Angiosarkom, Endotheliosarkom, Lymphangiosarkom, Lymphangioendotheliosarkom, Synoviom, Mesotheliom, Ewing-Tumor, Leiomyosarkom, Rhabdomyosarkom, Kolonkarzinom, Bauchspeicheldrüsenkrebs, Brustkrebs, Eierstockkrebs, Prostatakrebs, Plattenepithelkarzinom, Basalzellkarzinom, Adenokarzinom, Schweißdrüsenkarzinom, Talgdrüsenkarzinom, Zottenkarzinom, Zottenadenokarzinome, Cystadenokarzinom, Medullarkarzinom, bronchogenes Karzinom, Nierenzellkarzinom, Hepatom, Gallengangkarzinom, Choriokarzinom, Seminom, Embryonalkarzinom, Wilms-Tumor, Gebärmutterhalskrebs, Hodentumor, Lungenkarzinom, kleinzelliges Lungenkarzinom, Blasenkarzinom, Epithelkarzinom, Astrozytom, Medulloblastom, Kraniopharyngiom, Ependymom, Pinealom, Hämangioblastom, Akustikusneurinom, Oligodendrogliom, Meningiom, Neuroblastom, Retinoblastom; Leukämien, z. B. akute lymphatische Leukämie und akute myeloische Leukämie (Myeloblasten-, Promyelozyten-, Myelomonozyten-, Monozyten- und Erythroleukämie); chronische Leukämie (chronische Myelozyten- (Granulozyten)-leukämie und chronische Lymphozytenleukämie); und Polycythaemia vera, Lymphom (Hodgkin- und Non-Hodgkin-Lymphom), multiples Myelom, Waldenström-Makroglobulinämie und Schwere-Ketten-Krankheit.

5. Streptolysin O (SLO) zur Verwendung gemäß Anspruch 1, wobei das SLO dem Subjekt durch eine Verabreichungsart verabreicht wird, die aus der Gruppe ausgewählt ist, die intramuskulär, sublingual, intravenös, subkutan und intrathekal umfasst.

6. Streptolysin O (SLO) zur Verwendung gemäß Anspruch 5, wobei das SLO sublingual verabreicht wird.

7. Streptolysin O (SLO) zur Verwendung gemäß Anspruch 5, wobei das SLO subkutan verabreicht wird.

8. Streptolysin O (SLO) zur Verwendung gemäß Anspruch 1, wobei das SLO in einer Dosis von etwa 0,01 Einheiten bis etwa 10 Einheiten verabreicht wird.

9. Streptolysin O (SLO) zur Verwendung gemäß Anspruch 8, wobei das SLO in einer Dosis von etwa 1 Einheit bis etwa 5 Einheiten verabreicht wird.

10. Streptolysin O (SLO) zur Verwendung gemäß Anspruch 9, wobei das SLO in einer Dosis von etwa 2 Einheiten verabreicht wird.

11. Streptolysin O (SLO) zur Verwendung gemäß Anspruch 1, wobei das SLO mindestens zweimal täglich verabreicht wird.

12. Streptolysin O (SLO) zur Verwendung gemäß Anspruch 1, ferner beinhaltend das Verabreichen eines MMP-Hemmers an das Subjekt.

13. Streptolysin O (SLO) in Kombination mit einem MMP-Hemmer zur Verwendung beim Hemmen oder Unterdrücken von Krebsmetastasen bei einem Subjekt, beinhaltend das Verabreichen einer Kombinationstherapie an das Subjekt, die beinhaltet
(a) Steptolysin O (SLO) und
(b) einen MMP-Hemmer
wobei die Kombinationstherapie in einer Menge verabreicht wird, die wirksam ist, um Krebsmetastasen in dem Subjekt zu hemmen oder unterdrücken.

14. Streptolysin O (SLO) zur Verwendung beim Hemmen oder Unterdrücken von Metastasen einer Krebszelle durch In-Kontakt-Bringen der Krebszelle mit Streptolysin O (SLO) in einer Menge, die wirksam ist, um Metastasen der Krebszelle zu hemmen oder unterdrücken.

15. Streptolysin O (SLO) zur Verwendung gemäß Anspruch 14, wobei die Krebszelle aus einem krebsigen Gewebe stammt, das aus der Gruppe ausgewählt ist, die Gliom, Melanom, Fibrosarkom und Adenosarkom umfasst.

16. Streptolysin O (SLO) zur Verwendung gemäß Anspruch 15, wobei die Krebszelle aus einem krebsigen Gewebe stammt, das aus der Gruppe ausgewählt ist, die menschliche Sarkome und Karzinome umfasst.

17. Streptolysin O (SLO) zur Verwendung gemäß Anspruch 16, wobei die Krebszelle aus einem krebsigen Gewebe stammt, das aus der Gruppe ausgewählt ist, die umfasst: Myxosarkom, Liposarkom, Chondrosarkom, osteogenes Sarkom, Chordom, Angiosarkom, Endotheliosarkom, Lymphangiosarkom, Lymphangioendotheliosarkom, Synoviom, Mesotheliom, Ewing-Tumor, Leiomyosarkom, Rhabdomyosarkom, Kolonkarzinom, Bauchspeicheldrüsenkrebs, Brustkrebs, Eierstockkrebs, Prostatakrebs, Plattenepithelkarzinom, Basalzellkarzinom, Adenokarzinom, Schweißdrüsenkarzinom, Talgdrüsenkarzinom, Zottenkarzinom, Zottenadenokarzinome, Cystadenokarzinom, Medullarkarzinom, bronchogenes Karzinom, Nierenzellkarzinom, Hepatom, Gallengangkarzinom, Choriokarzinom, Seminom, Embryonalkarzinom, Wilms-Tumor, Gebärmutterhalskrebs, Hodentumor, Lungenkarzinom, kleinzelliges Lungenkarzinom, Blasenkarzinom, Epithelkarzinom, Astrozytom, Medulloblastom, Kraniopharyngiom, Ependymom, Pinealom, Hämangioblastom, Akustikusneurinom, Oligodendrogliom, Meningiom, Neuroblastom, Retinoblastom; Leukämien, z. B. akute lymphatische Leukämie und akute myeloische Leukämie (Myeloblasten-, Promyelozyten-, Myelomonozyten-, Monozyten- und Erythroleukämie); chronische Leukämie (chronische Myelozyten- (Granulozyten)-leukämie und chronische Lymphozytenleukämie); und Polycythaemia vera, Lymphom (Hodgkin- und Non-Hodgkin-Lymphom), multiples Myelom, Waldenström-Makroglobulinämie und Schwere-Ketten-Krankheit.

## Revendications

1. Streptolysine 0 (SLO) pour utilisation afin d'empêcher une métastase de cancer chez un sujet, comprenant l'administration de streptolysine 0 (SLO) au sujet en quantité efficace pour empêcher une métastase de la tumeur.

2. Streptolysine 0 (SLO) pour utilisation selon la revendication 1, dans laquelle le cancer est sélectionné dans le groupe constitué d'un gliome, d'un mélanome, d'un fibrosarcome et d'un adénosarcome.

3. Streptolysine 0 (SLO) pour utilisation selon la revendication 1, dans laquelle le cancer est sélectionné dans le groupe constitué des sarcomes et des carcinomes humains.

4. Streptolysine 0 (SLO) pour utilisation selon la revendication 3, dans laquelle le cancer est sélectionné dans le groupe constitué des suivants : myxosarcome, liposarcome, chondrosarcome, sarcome ostéogénique, chordome, angiosarcome, endothéliosarcome, lymphangiosarcome, lymphangioendothéliosarcome, synovialome, mésothéliome, sarcome d'Ewing, léiomyosarcome, rhabdomyosarcome, cancer du colon, cancer du pancréas, cancer du sein, cancer des ovaires, cancer de la prostate, cancer de l'épithélium malpighien, épithélioma cutané basocellulaire, adénocarcinome, cancer des glandes sudoripares, cancer des glandes sébacées, carcinome papillaire, adénocarcinomes papillaires, cystadénocarcinome, cancer médullaire, cancer broncho-pulmonaire, cancer des cellules rénales, hépatome, cancer des voies biliaires, choriocarcinome, séminome, carcinome embryonnaire, tumeur de Wilms, cancer des cervicales, tumeur testiculaire, cancer du poumon, cancer pulmonaire à petites cellules, cancer de la vessie, cancer épithélial, astrocytome, médulloblastome, craniopharyngiome, épendymome, pinéalome, hémangioblastome, neurome acoustique, oligodendrogliome, méningiome, neuroblastome, rétinoblastome ; leucémies, par exemple la leucémie lymphocytaire aiguë et la leucémie myélocitaire aiguë (leucémie myéloblastique, promyélocytaire, myélomonocytaire, monocytaire et monomyélocytaire) ; la leucémie chronique (leucémie myélocytaire chronique (granulocytaire) et la leucémie lymphocytaire chronique) ; et polycythémie vraie, lymphome (maladie de Hodgkin et maladie de type non hodgkinien), myélome multiple, macroglobulinémie de Waldenstrom et maladie des chaînes lourdes.

5. Streptolysine 0 (SLO) pour utilisation selon la revendication 1, dans laquelle la SLO est administrée au sujet par une voie sélectionnée dans le groupe constitué des voies intramusculaire, sublinguale, intraveineuse, sous-cutanée et intrathécale.

6. Streptolysine 0 (SLO) pour utilisation selon la revendication 5, dans laquelle la SLO est administrée par voie sublinguale.

7. Streptolysine 0 (SLO) pour utilisation selon la revendication 5, dans laquelle la SLO est administrée par voie sous-cutanée.

8. Streptolysine 0 (SLO) pour utilisation selon la revendication 1, dans laquelle la SLO est administrée en dose d'environ 0,01 unité à environ 10 unités.

9. Streptolysine 0 (SLO) pour utilisation selon la revendication 8, dans laquelle la SLO est administrée en dose d'environ 1 unité à environ 5 unités.

10. Streptolysine 0 (SLO) pour utilisation selon la revendication 9, dans laquelle la SLO est administrée en dose d'environ 2 unités.

11. Streptolysine 0 (SLO) pour utilisation selon la revendication 1, dans laquelle la SLO est administrée au moins deux fois par jour.

12. Streptolysine 0 (SLO) pour utilisation selon la revendication 1, comprenant en outre l'administration d'un inhibiteur de MMP audit sujet.

13. Streptolysine 0 (SLO) en combinaison avec un inhibiteur de MMP pour utilisation afin d'empecher ou de supprimer une métastase de cancer chez un sujet comprenant l'administration audit sujet d'une thérapie combinée, comprenant :
(a) de la streptolysine 0 (SLO) et
(b) un inhibiteur de MMP,
dans laquelle la thérapie combinée est administrée en quantité efficace pour empêcher ou supprimer une métastase de cancer chez le sujet.

14. Streptolysine 0 (SLO) pour utilisation afin d'empêcher ou de supprimer une métastase de cellule cancéreuse en mettant en contact la cellule cancéreuse avec de la streptolysine 0 (SLO) en quantité efficace pour empêcher ou supprimer la métastase de la cellule cancéreuse.

15. Streptolysine 0 (SLO) pour utilisation selon la revendication 14, dans laquelle la cellule cancéreuse provient d'un tissu cancéreux sélectionné dans le groupe constitué d'un gliome, d'un mélanome, d'un fibrosarcome et d'un adénosarcome.

16. Streptolysine 0 (SLO) pour utilisation selon la revendication 15, dans laquelle la cellule cancéreuse provient d'un tissu cancéreux sélectionné dans le groupe constitué des sarcomes et des carcinomes humains.

17. Streptolysine 0 (SLO) pour utilisation selon la revendication 16, dans laquelle la cellule cancéreuse provient d'un tissu cancéreux sélectionné dans le groupe constitué des suivants : myxosarcome, liposarcome, chondrosarcome, sarcome ostéogénique, chordome, angiosarcome, endothéliosarcome, lymphangiosarcome, lymphangioendothéliosarcome, synovialome, mésothéliome, sarcome d'Ewing, léiomyosarcome, rhabdomyosarcome, cancer du colon, cancer du pancréas, cancer du sein, cancer des ovaires, cancer de la prostate, cancer de l'épithélium malpighien, épithélioma cutané basocellulaire, adénocarcinome, cancer des glandes sudoripares, cancer des glandes sébacées, carcinome papillaire, adénocarcinomes papillaires, cystadénocarcinome, cancer médullaire, cancer broncho-pulmonaire, cancer des cellules rénales, hépatome, cancer des voies biliaires, choriocarcinome, séminome, carcinome embryonnaire, tumeur de Wilms, cancer des cervicales, tumeur testiculaire, cancer du poumon, cancer pulmonaire à petites cellules, cancer de la vessie, cancer épithélial, astrocytome, médulloblastome, craniopharyngiome, épendymome, pinéalome, hémangioblastome, neurome acoustique, oligodendrogliome, méningiome, neuroblastome, rétinoblastome ; leucémies, par exemple, leucémie lymphocytaire aiguë et leucémie myélocitaire aiguë (leucémie myéloblastique, promyélocytaire, myélomonocytaire, monocytaire et monomyélocytaire) ; leucémie chronique (leucémie myélocytaire chronique (granulocytaire) et leucémie lymphocytaire chronique) ; et polycythémie vraie, lymphome (maladie de Hodgkin et maladie de type non hodgkinien), myélome multiple, macroglobulinémie de Waldenström et maladie des chaînes lourdes.
